# EUROPEAN PATENT SPECIFICATION

(11) **EP 3 536 300 B1**
(45) Date of publication and mention of the grant of the patent: **12.06.2024**
(21) Application number: 17866942.0
(22) Date of filing: 03.08.2017
(51) Int. Cl.: A61J 7/04, A61B 5/00, A61B 5/282, A61B 5/30, A61B 5/0245, A61B 5/024

(54) **WEARABLE ELECTROCARDIOGRAPHY (ECG) MONITORING TECHNOLOGY WITH SEALED TANK FOR MEDICATION AND INTEGRATED MEDICAL MONITORING SYSTEM**
TRAGBARE ELEKTROKARDIOGRAFIE (EKG)-ÜBERWACHUNGSTECHNOLOGIE MIT ABGEDICHTETEM TANK ZUR MEDIKATION UND INTEGRIERTES MEDIZINISCHES ÜBERWACHUNGSSYSTEM
TECHNOLOGIE PORTABLE DE SURVEILLANCE ÉLECTROCARDIOGRAPHIQUE (ECG) AVEC RÉSERVOIR HERMÉTIQUE POUR MÉDICAMENTS ET SYSTÈME DE SURVEILLANCE MÉDICALE INTÉGRÉ

(30) Priority: 07.11.2016 BR 102016025939
(43) Date of publication of application: 11.09.2019
(73) Proprietor: Lótus Medicina Avançada, Minas Gerais (BR)
(72) Inventor: CASTAGNA, Ilene Maria Guimarães de Siqueira, 35460-000 Brumadinho (BR); CASTAGNA, Marco Túlio Vilaça, 35460-000 Brumadinho (BR)
(74) Representative: Studio Torta S.p.A.
(86) International application number: PCT/BR2017/050219
(87) International publication number: WO 2018/081884

(56) References cited:
- WO-A1-2015/179015
- WO-A2-2012/088398
- US-A1- 2013 281 815
- US-A1- 2014 364 779
- US-A1- 2015 272 464
- US-A1- 2016 066 809
- US-B1- 6 304 797
- US-B1- 6 304 797
- US-B1- 6 970 731
- YANG GENG ET AL: "A Health-IoT Platform Based on the Integration of Intelligent Packaging, Unobtrusive Bio-Sensor, and Intelligent Medicine Box", IEEE TRANSACTIONS ON INDUSTRIAL INFORMATICS, IEEE SERVICE CENTER, NEW YORK, NY, US, vol. 10, no. 4, 1 November 2014 (2014-11-01), pages 2180-2191, XP011563699, ISSN: 1551-3203, DOI: 10.1109/TII.2014.2307795 [retrieved on 2014-11-04]
- UPKAR VARSHNEY: "Wireless Medication Management System: Design and performance evaluation", WIRELESS TELECOMMUNICATIONS SYMPOSIUM (WTS), 2011, IEEE, 13 April 2011 (2011-04-13), pages 1-8, XP031903510, DOI: 10.1109/WTS.2011.5960858 ISBN: 978-1-4577-0162-7

## Description

The present invention refers to a wearable, individual, customized and different sized technology for men and women, composed of electrodes, electronic circuit and airtight container for medicines, coupled to one another, and a mini electrocardiogram (ECG) apparatus containing a GSM (Global System Mobile) modem and a GPS (Global Positioning System) or a Bluetooth system which, through a wireless network specification within a personal scope (Wireless Personal Area Networks - PANs) deemed as PAN-type or even WPAN, provides a way to connect to and exchange information between the ECG device in the system and the mobile phones (smartphones) utilizing a specific application (app). The acquisition of electrical signals begins when the user presses the button down.

The present invention is in the field of the medical applications, aiming at monitoring patients at high cardiovascular risk, being possible to diagnose it as soon as possible, aiming at shortening time to definite treatment of those who present acute coronary syndrome (ACS), acute myocardial infarction (AMI), acute atrial fibrillation-type (AAF) cardiac arrhythmias, and other cardiac arrhythmias or other cardiac pathologies detectable by electrocardiographic trace, by analyzing a bunch of data - big data analytics and deep learning - by utilizing artificial intelligence, utilizing the internet of things, as well as for recreational and/or sport applications, aiming at monitoring individuals who desire their electrocardiographic trace to be followed up during their physical, recreational or sportive activities, being possible to evaluate their performance in a single or evolutionary manner, or even to have their electrocardiogram recorded and written.

The ECG apparatus captures electronic signals by the electrodes, allowing for a remote electrocardiogram monitoring. The data collected by the ECG and the location by the GPS are transferred online via GSM or Bluetooth to a user's mobile phone (smartphone), which has an app, and from this app to a datacenter having an Integrated Monitoring System (IMS) in a secure and reliable manner, with a 24 x 7 x 365 coverage. The GPS of the ECG apparatus or of the smartphone itself, in the case of transmission via Bluetooth to the smartphone, enables to locate the individual who is wearing the ECG system according to this invention. The compartments for medicines are integrated to the electronic circuit of the ECG apparatus, and remotely controlled.

This hardware- and software-integrating system allows for the transmission of low voltage electronic signals through Internet of Things to an integrated monitoring center, as well as create an interface which enables to shorten time since early symptoms that may be a sign of an ACS, or an AMI, or an AAF, or another cardiac arrhythmia, even the definite treatment, which may also anticipate the beginning of treatment, from remote dispensation of the medicines available in the plastic box of medicines coupled to the box of mini ECG.

The coronary artery disease (CAD) is the main cause of death worldwide. The acute myocardial infarction (AMI) is the complete occlusion of coronary artery, vessel that irrigates the cardiac muscle. The best treatment option is the primary angioplasty: unclogging of the coronary artery through balloon catheter or stent. The earlier the coronary is opened the best will be the result of the treatment, therefore reducing the morbidity and mortality rates. The maximum optimal time among entry of the individual to emergency (door), diagnose and treatment is up to 90 min. The main metric in the treatment of AMI is Door to Balloon time: "D2B", which is the time from patient's arrival at emergency to his/ her coronary opening by primary angioplasty with a balloon catheter or stent.

It is perceivable the need for the patient to be more autonomous, have information and be capable of using resources such as telehealth, for obtaining analysis, diagnoses and even to begin his/ her treatment regardless of the location. By means of continuous technological advance and computing and mobile communication facilities, it is proposed to widen the limits of this paradigm D2B in the AMI, the ACS and in the AAF approach, in addition to other cardiac arrhythmias.

All data generated by the technology coupled to the system claimed in this present invention is based on monitoring center, hospital network and expert medical doctor. Through an electronic signal sent by the monitoring center, the doctor responsible for the analysis of the electrocardiographic trace provides the diagnose and remotely triggers, if necessary, the medicine compartment opening, thereby managing to start treatment early and also reducing the time from the first symptoms in patient with a clogged coronary artery to the effective and definite time that is the opening of the coronary artery by primary angioplasty. The same process may be carried out with regard to cardiac arrhythmia, such as AAF, the symptoms having got started less than 24 to 48 hours, providing a definite treatment with proper medicine dispensing by remote opening of the compartments with medicines as soon as the patient is diagnosed.

Individuals having CAD are instructed to monitor themselves having a support of telemedicine to speed up finding their symptoms. Thus, the significant reduction in the time from the first symptoms to the opening of the coronary artery will occur through a combination of a remote monitoring network that generates reports and supports patients at AMI and ACS risk, popularizing the use of the system. It may also occur with patients presenting AAF-type arrhythmia, from the moment they feel a cardiac rhythm change beginning in a period of time up to 48 hours.

The development of wearable technologies and Internet of Things provide several possibilities of application in healthcare, such as remote monitoring of individuals at cardiovascular risk and seniors. With the available technology, in addition to preventing programs through educational initiatives, healthcare opportunities may be provided.

Data collected by the ECG and its location by the GPS (Global Positioning System) are transferred online via GSM (Global System Mobile) or via Bluetooth coupled to the electrocardiogram apparatus to the smartphone containing an app, and from this one to a datacenter with an Integrated Monitoring System (IMS). This IMS will assign the received ECG data to a doctor, who will remotely provide the ECG report by electronic means.

The hospital network and the doctors capable of providing reports of the monitored patients will previously be registered in the IMS. When AMI or ACS is identified, the team acting on IMS will assist the patient, indicating first procedures and forward names and addresses of nearby hospitals able to take care of the patient. When identified AAF or other cardiac arrhythmias, by the registered doctor, the patient will be instructed to take the specific medicine for this pathology after remote opening of the plastic box containing proper drug by means of an electronic signal.

It was found out documents presenting technologies regarding to clothes with coupled ECG or GSM or GPS or Bluetooth technologies, however, without specific functionality of utilization of the complex monitoring system integrated to the patient, as well as access to the airtight plastic box containing medicines for quick use with remote opening through encrypted digital signature of an expert doctor.

The document "Elastic garment for positioning and fixing ECG electrodes" (US20160066809) only describes a cloth having position marks and electrodes fixation, but it does not describe a fabric providing a tensile strength over body, such that the electrodes are close and tight to the skin. They are horizontally and vertically embroidered by crossing one another, with stainless steel yarns isolated or associated to the silver- or carbon-based dyes or pastes or conductive stretchable textiles over the cloth fabric, so as to form a mesh to widen the surface contacting the underlying skin. No reference is made to any device containing airtight container for medicines that enables remote dispensing of medicines, in case of specific needs such as ACS, AMI, AAF and other cardiac arrhythmias, for early treatment to these pathologies.

The document "Wearable remote electrophysiological monitoring system" (US20130281815) consists of an electrophysiological monitoring system that record ECG data which is coupled to a cloth. It presents a wireless transmitter and controller configured to collect data generated by the ECG, and in the case of a warning issued to a receiver due to any abnormality. The document "Multi-layered sensor apparatus" (WO2012088398) describes only a cloth formed by an interconnected sensor array without, however, demonstrating that these sensors are part of an ECG apparatus for continuous or intermittent monitoring, and no document describes the functionality of remote ECG monitoring in real time, and also does not make reference to any device containing airtight container for medicines enabling remote dispensing of medicines.

The document "Wearable medical treatment device" (EP2590710) makes reference to a wearable technology including a cardiac detection electrode, a treatment electrode, a user interface configured to receive information, and a sensor configured to sense some abnormal physiological activity. But it neither demonstrates a complete ECG apparatus nor makes reference to any type of an intermittent or system-integrated monitoring, remote or not. It does not refer to any remote control device for opening an airtight plastic box containing medicines suitable for quick start using an encrypted digital signature.

The document "ECG method and system for optimal cardiac disease detection" (US20070219454) describes a cardiac disease indicator method through electrocardiogram signals from a set of leads fixed to the patient's body for detecting and mapping cardiac diseases in the patient's body, and the document "Wireless transmitted electrocardiogram monitoring device" (US7412281) only refers to a wireless electrocardiogram monitoring system for monitoring of long-term physical state. None of these documents makes reference to a wearable technology with complex functionalities of remote data mapping and transmission to a monitoring center, as well as access to a treatment of cardiac disease, when necessary, by means of opening of an airtight plastic box containing medicines as previously describes.

The documents "Apparatus for, and method of, determining the condition of a patient's heart" (EP1673010), and "Ambulatory physiological evaluation system including cardiac monitoring" (WO8902246) describe a vest adapted to be used over the patient's upper body, and a system of ambulatory evaluation to monitor the cardiac activities of a patient, but not comprising the whole data intermittent and remote reading integrated monitoring system of the patients, having possibility of immediate service and immediate treatment.

In turn, the document "Independent non-interfering wearable health monitoring and alert system" (CA2839954) describes a cloth with a system including a multi-ECG metering device having alert markers from changes and detection of health abnormality, the yellow level serving as an indicator for a medical check, and the red level serving as an alert to seek for immediately medical help. However, it does not present a specific functionality of utilization of complex monitoring system integrated to the patient, by which warnings are issued to a specific center that triggers a complete remote service, identifying the service location by the apparatus GPS coupled to the cloth, then the responsible doctor will, by remote access and encrypted digital signature, open an airtight plastic box containing medicines immediately start the treatment until an ambulance arrives and the patient is properly cared for in a hospital or other suitable location.

The document "A Health-IoT Platform Based on the Integration of Intelligent Packaging, Unobtrusive BioSensor, and Intelligent Medicine Box" (DOI 10.1109/TII.2014.2307795) presents an intelligent home-based platform that fuses IoT devices, such as wearable sensors and medical monitoring systems, with in-home healthcare services, such as telemedicine. This platform aims to optimize financial costs and hospitals' resources by allowing an optimized remote medical care for routine and low severity cases. It has a real-time monitoring and analyzing capability of the patient's health status, identifying early symptoms of any life-threatening events, and tracks the patient's treatment. The Health-IoT System from this document is comprised by an intelligent medicine box that tracks the necessary dosage of each medicine and send audio or video reminders to which kind of medicine to take, and how much of it the patient should take. The box can identify through a short-range RFID which medicine was taken and the amount, but it does not control the dosage that is released.

The document "Automated Medication Dispenser with Remote Patient Monitoring System" (US6304797) describes a dispensing unit with a plurality of compartments, each compartment adapted to store a dose of medication. The invention of US6304797 comprises a garment, a monitoring device, and a medication-dispensing unit. The garment includes at least one sensor, a torso portion adapted to fit the torso of a patient and defining at least one aperture to house the sensor, and a connector communicating with the sensor. Either the torso portion or the arm portion defines a one channel linking the connector to the sensor, where said channel houses a signal transmission conduit that couples the sensor to the connector. The sensors contained within the garment presented have the function to locate the patient, with the possibility to also collect vital signs, and are positioned properly for each patient individually. The system is connected to a patient monitoring unit, located by the patient's bedside. The system of document US6304797 is developed for use in closed and controlled spaces, such as hospital beds and in-home care situations, and does not present or suggest the use of its system in an outside condition, for sportive, recreational or even medication situations, where the patient can move freely, and all communication is made through Bluetooth or GSM.

None of the documents found out in the state of art describes or alludes similar technology, covering the IoT technology, the medicine container with controlled distribution of medicine and dosage, mini ECG sensors properly disposed on the patient's body, either on a cloth or directly over the skin, and the communication via Bluetooth and/or GSM with a doctor, especially for an outside use for a freely moving patient, considering also recreational and sportive activities. Also, none presents performance, efficiency and integration of equipments similar to those of the present invention, as well as complex functionalities of mapping and transmission of remote data to a monitoring center, utilizing artificial intelligence, "deep learning" and big data analytics, as well as access to early treatment of the coronary artery disease, either AMI or ACS, AAF, in a period of time less than 48 hours, or other cardiac arrhythmias, when necessary, by means of opening of the airtight plastic box containing medicines as previously described, by remote transmission of electronic signal, authorized and carried out by the doctor who provides the ECG report.

### SUMMARY OF THE INVENTION

The present system comprises a wearable system according to claim **1.** Embodiments are defined in claims **2-4.**

The airtight compartment is coupled to the electronic circuit of the ECG system with GSM, GPS or BLE, and is controlled by a solenoid and an electromechanical system. Thus, the expert doctor, with an encrypted digital signature, can remotely trigger the opening of the doors of the airtight compartment containing medicines for proper dispensing, after the diagnostic confirmation.

The remote monitoring of the electrocardiogram has the optional function of automatic interpretation of the trace to be carried out by ECG analysis software, supported by an expert doctor and an expert system, a software of Integrated Monitoring System (IMS) online and a software integrated to the smartphone receiving the electronic signals that enables:
- the electronic signals captured by the electrocardiogram to transmit the trace and be visible on the smartphone in real time, via BLE transmission coupled to the electrocardiogram for that smartphone; and
- recording to be made for a period of 24 hours of that tracing.

In addition, on-screen technology allows the self-monitoring of symptoms by patients themselves, who can have their treatment instituted as soon as the symptoms start, and their ECG is remotely done.

The application-based communication of screen technology takes place as follows:
- the user accesses data via the smartphone, by GSM or BLE, in real time via the application, in addition to access the hardware with encompassed software, which performs the acquisition of the electrocardiographic signals;
- the hardware with encompassed software has direct communication with the smartphone;
- the smartphone communicates via Wi-Fi, 3G, 4G or 5G transmission with the integrated monitoring center and a second smartphone;

- the second smartphone is accessed by a doctor or an expert.
- the integrated monitoring center communicates with a hospital or similar treatment center and with the doctor or expert via a second smartphone or computer.

In addition, the wearable technology is comprised of polyamide textile yarn fabric or any fabrics similar to synthetic and expandable rubbers which can provide skin protection against the harmful effects of the sun, offer bacteriostatic action, avoiding body odor, and be biodegradable.

Additionally, the wearable technology enables the electrodes to have stainless steel yarns horizontally and vertically embroidered by crossing or meshing one another on the cloth, so as to form a mesh to widen the surface contacting the underlying skin, in a rectangular or square shape, with conductivity property of electronic signals or composed of silver- or carbon-based pastes or dyes or stretchable conductive fabrics, in a single manner or associated to the mesh of stainless steel yarns, singly or associated to a silver- or carbon-based dye or pastes or stretchable conductive fabrics, as well as for printing low voltage electronic circuits over elastic films and fabric substrates, and contains stretchable encapsulating which is used to protect the low voltage circuit printed over the elastic film and substrate fabric.

Data collected by the ECG system and its location by the GPS are transferred online via GSM or by integrating BLE to the smartphone that contains an application (app), for a datacenter with an Integrated Monitoring System (IMS). Thus, said IMS will assign the received ECG data to a professional who will immediately and remotely provide the ECG report to the users, indicating the first procedures, allowing immediate interaction with an emergency hospital medical service and forwarding the names and addresses of nearby hospitals able to serve.

The data storage is carried out by the electronic circuit U3, non-volatile memory responsible for recording the acquired data for later transmission, and the circuit U8 is composed of a solid-state relay responsible for the activation of the electromagnet to release the compartment for the medicines. In addition, there is an application request for USB input for protection against outbreaks and electrostatic discharges, formed by the U7 circuit, solid state TVS protector.

The method (no method is forming part of the claims) for acquiring electrocardiographic signals acquires 256 points per second, where the signals are acquired by an ECG device contained in the system, and the ECG device acquires the signals in a period of time of at most 24 hours. The variation of the electrical potentials generated by the electrical activity in the heart reads out 8 channels, where 4 additional channels are generated by means of mathematical calculations carried out on the 8 read channels, and the device requires up to 8 seconds to set the impedance for each type of skin.

The method (no method is forming part of the claims) for acquiring electrocardiographic signals also uses an application (app) installed on the smartphone that adds a timestamp and the acquired GPS coordinate; submits the examination to the FTP server as a file form, in addition to be able to view both the recorded exam and the monitoring in real time of the cardiac signals. In case the file is not successfully send, the application stores an error log, and a new attempt takes place, and the process is repeated until the file is successfully submitted to the server, and the file is afterwards sent to the central along with the next valid exam.

The on-screen hardware is powered by a rechargeable LI-ION battery containing LEDs and a buzzer to notice the patient the stage of the examination process and battery status, a BLE with antenna, and an USP input for programming and setting up the device.

The on-screen system also enables the electrodes to be fixed directly over the skin of the patient instead of directly on the vest.

### BRIEF DESCRIPTION OF FIGURES

For better understanding on how the invention herein claimed is constituted, the appended drawings are presented, wherein:
Figure 1 - analog Front-end, responsible for scanning biometric data.
Figure 2 - GPS, responsible for acquiring geographical coordinates.
Figure 3 - CPU and GRPS Module, responsible for processing and transmitting data.
Figure 4 - wearable individual, customized and different sized technology (1), for men and women, the integrated system being composed of conductive track (2), airtight container for medicines (3) and electrodes (4), coupled to one another, and an electrocardiogram (ECG) apparatus.

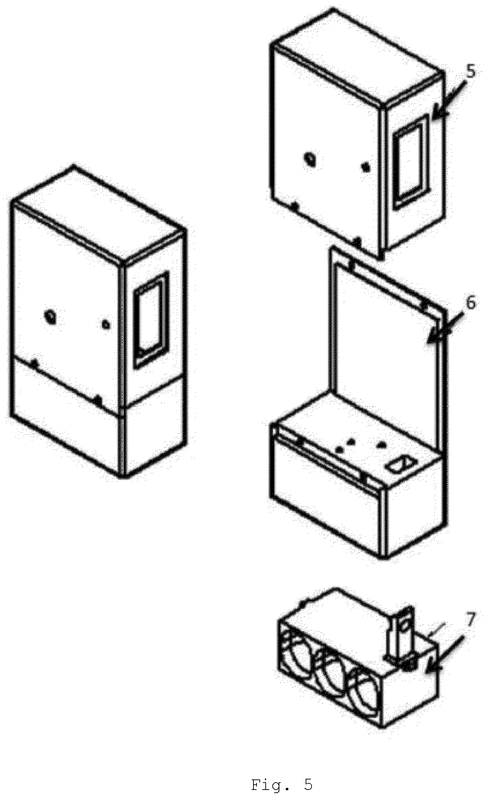

Figure 5 - airtight container for medicines, the lower compartment (5) being to fit electronic pieces, the upper compartment (6) being to fit electronic pieces, and a medicine compartment (7).

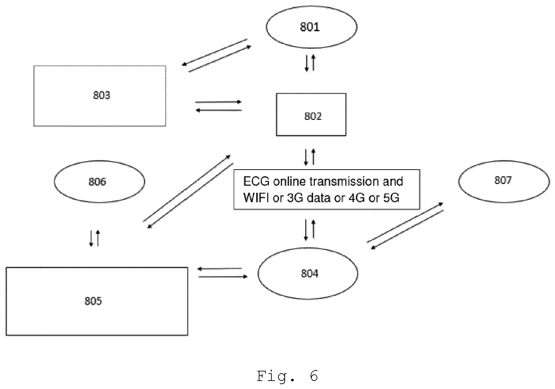

Figure 6 - communication system of the on-screen technology.

### DETAILED DESCRIPTION OF THE TECHNOLOGY

### DESCRIPTION OF THE CLOTH QUALITY

The cloth (1) is composed of a fabric of polyamide textile yarns 6.6, providing great comfort, lightness, softness, care easiness and functionality. Specifically, yarns are made which may i) offer protection to the skin against harmful effects of sun, ii) have bacteriostatic action, preventing body odor, iii) be biodegradable, allowing the clothes made therefrom to be decomposed less than 3 years, when correctly discarded on landfills.

These fabrics provide a tensile strength over body, such that the electrodes (4) are close and tight to the skin. The cloth electrodes (4) are composed of a conductive track (2) composed of horizontally and vertically embroidered stainless steel yarns crossing one another over the cloth fabric, in a single manner or associated to silver- and/or carbon-based dye or paste or fabric, so as to form a mesh to widen the surface contacting the underlying skin in a rectangle or square shape about 5 × 5 cm² or 5 × 4 cm² or 4 × 4 cm² or 5 × 6 cm² or 6 × 6 cm² or 6 × 5 cm² or 6 × 4 cm². The electrodes (4) of the cloth composed of stainless-steel yarn embroidered in a single manner or associated to a silver- and/or carbon-based dye or paste or fabric have a property of electrical signals' conductivity. The "trace" conducting the electrical sign sensed by the embroidered electrodes contacting the skin comprises different yarns resulting from the stainless-steel yarns embroidered, associated or not to the silver- and/or carbon-based dye or paste or fabric conducting electrical signal from the embroidered electrode (4) to the connection of the electrocardiographic device. The electrodes (4) of the fabric are composed of stretchable silver- and/or carbon-based conductive pastes, dyes or fabrics to print low voltage electric circuits over elastic films and textile substrates, having excellent stretchability, gripping capability and conductibility. These conductive textiles, dyes or pastes contain an encapsulating stretchable component which is used to protect the low voltage circuit printed onto the elastic film and the substrate textile, since they have an excellent thin and stretchable protection, gripping property and abrasion resistance. This system may be designed to wearable electronic applications that may require repeatedly washing.

The device encompasses the variation of electric potentials generated by electrical activity of the heart. 8 channels are read out, and other 4 channels are generated by means of mathematical calculations carried out on the 8 read channels. The acquisition of electrical signals starts when the patient presses down the button, acquiring 256 points per second during a span of 15 seconds, taking about 8 seconds to set the impedance of each skin type. The read-out values are stored in a NOR memory, until they can be sent to a server by means of a GPRS connection, meanwhile the GPS starts to synchronize satellites and identify the patient's location, until receive coordinates or the limit of time is exceeded.

The wearable embodiment of the ECG monitoring technology (1) is individual, customized, is differently sized, and for men and women, in addition to be composed of a hardware, having 12 bypasses and an encompassed software with a feature of GPS access with interface for SIM card for GSM communication or through Bluetooth coupled to the ECG. This system will transmit the electronic signal of the ECG to an Analysis Software Module (ASM), generating graphics, thus allowing for a remote electrocardiogram monitoring, with optional function of automatic interpretation, coupled to a high technology of analysis of a bunch of data and utilizing artificial intelligence, big data analytics and deep learning. All data generated by the technology coupled to the cloth claimed in the present invention are based on monitoring center, hospital network and expert doctor.

The capture of the electronic signals by the electrodes allows for a remote monitoring of electrocardiogram, with optional function of automatic interpretation, supported by an expert system, a software of Integrated Monitoring System (IMS) online that are the interfaces with doctors, hospitals and clients integrating with SMS Broker.

After reading out the signals, the exam is sent to the server along with the coordinates of the place where the device was at the time of sending. If the file is not successfully sent, an error log is stored, and a new attempt is made after a time x, this is repeated until the exam can be submitted to the server. The log will be sent to the remote monitoring center along with the next valid exam. The equipment contains two LEDs and a buzzer to notify the patient about the stage of the examination process.

The data collected by the ECG and its location collected by the GPS will be transferred online via GSM or via Bluetooth to the smartphone and from this one to a datacenter with an Integrated Monitoring System (IMS) with a safe and reliable 24 × 7 × 365 coverage. This IMS will assign the received ECG data to a doctor who will immediately provide the ECG report electronically.

The device is coupled to an airtight compartment (3) containing specific medicines which are essential for the immediate start of the treatment of pathologies, such as ACS, AMI, AAF and other cardiac arrhythmias, integrated into the electronic circuit of the ECG apparatus controlled by a solenoid. The patient, stricken with ACS, AMI, FAA and other cardiac arrhythmias, may get the treatment started as soon as the symptom starts, and his ECG is remotely done by self-monitoring.

The expert doctor, through an encrypted digital signature, will remotely trigger the opening of the compartment doors containing medicines for proper remote dispensing, according to the diagnosis of the electrocardiographic trace. The server will identify the problem during the analysis, and remotely release access to the drug. The patient will receive a call informing that the drug has been released and then can press the button down for 5 seconds. The device, on the other hand, finds out the release file on the server and releases access to the medicine so it can be ingested. The server will have in its database all contacts of registered clients and doctors.

### FUNCTIONAL DESCRIPTION OF THE CIRCUIT

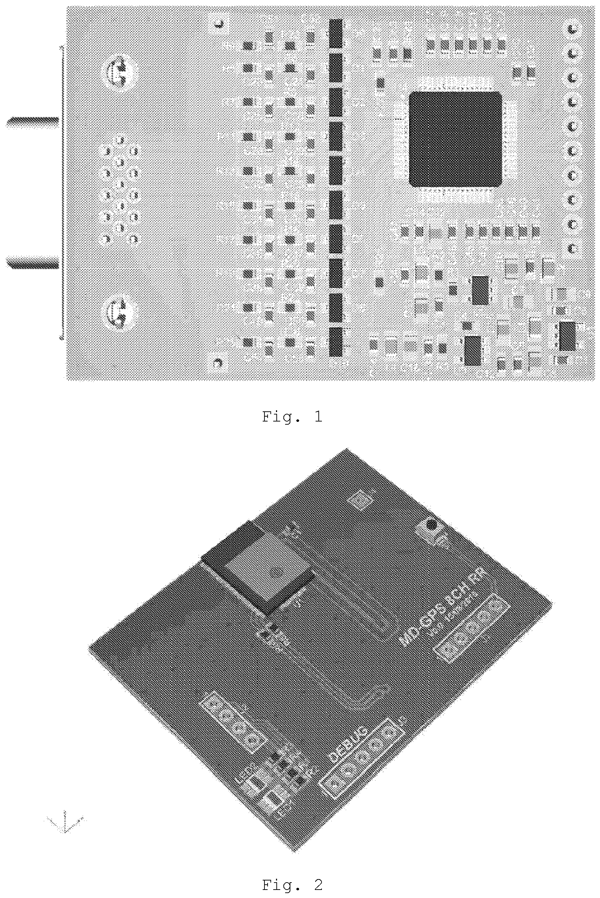

### ANALOG FRONT-END (FIGURE 1)

The biometric (analog) data are read out through the input connector J1, the signals pass through a high frequency filter formed by the components: R9, R5, R7, R11, R13, R15, R17, R19, R21, R25, R26, R22, R20, R18, R16, R14, R12, R8, R6, R23, C51, C52, C23, C24, C25, C26, C28, C29, C32, C33, C34, C35, C36, C37, C38, C3, C49, C50, C54, and C55.

The protection against electrostatic discharges at the input of the signals is made by the diodes D1 to D9, responsible for keeping the levels within the levels accepted by the A/D converter. Signal acquisition is performed by the U4 analog front-end, which consists of 8 24-bit delta-sigma A/D converters. The +2.5-Volt and -2.5-Volt symmetric voltage levels, required for the analog front-end signal levels to be suitable, are generated by the electronic circuits U1, U2 e U3.

### GPS COORDINATE ACQUISITION MODULE, SIGNALING AND TRIGGERING BUTTON (FIGURE 2)

For the acquisition of geographical coordinate data, we use a GPS module formed for electronic circuit U1, delivering data to the processing unit in the NMEA 0183 format, having latitude and longitude data. The LED1 and LED2 components are responsible for the operational signaling of the equipment and the key S1 to start up operating.

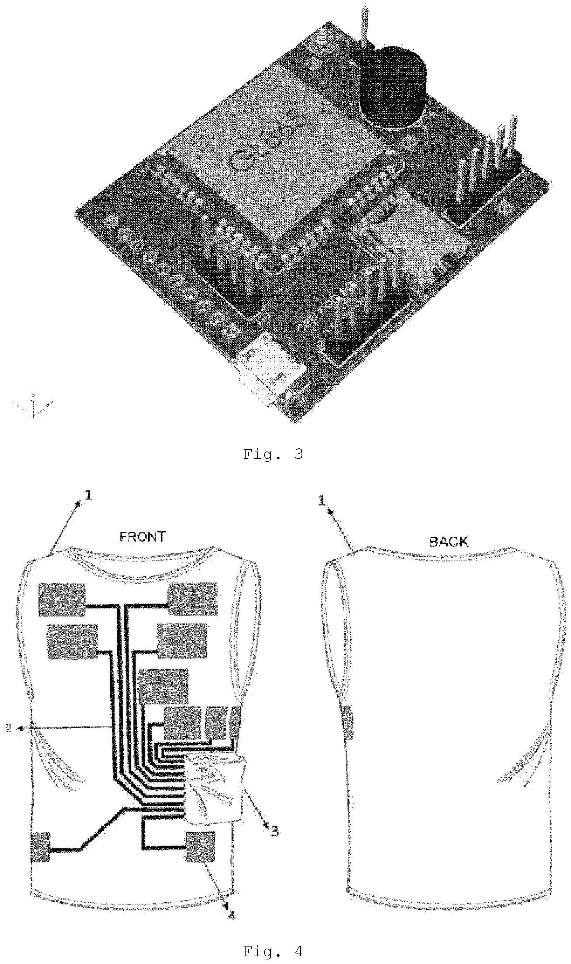

### PROCESSING UNIT AND GPRS MODULE (FIGURE 3)

The circuit is powered by a rechargeable LI-ION battery whose management is the responsibility of the U1 electronic circuit, front-end dedicated battery charger. The electronic circuit U2 aims at regulating the voltage levels to + 3.3 V required for the operation of the other electronic circuits.

Data storage is performed by the electronic circuit U3, non-volatile memory, responsible for recording acquired data for later transmission. The circuit U8 is composed by a solid-state relay responsible for the electromagnet triggering in order to release the compartment for medicines, according to the application request. For USB input, there is a protection against outbreaks and electrostatic discharges formed by the circuit U7, a solid-state TVS protection.

The communication via GPRS is carried out by the electronic circuit U6, Quad-Band GSM modem, responsible for sending data to the FTP server. The circuit U5 is composed by a solid-state relay, responsible for disconnecting the GPRS Modem when it is not operating.

The connector J6 is a receptacle for GSM SIMCARD which has its own protection against electrostatic outbreaks carried out by the electronic circuit TVS1. The data processing and treatment are responsibility of the electronic circuit U4, a microcontroller of the ARM family, responsible for executing the application software.

### INTEGRATION OF MINI ECG-BLUETOOTH AND SMARTPHONE

The device acquires the variation of electrical potentials generated by the electrical activity of the heart. 8 channels are read out, and other 4 channels are generated by means of mathematical calculations carried out on the 8 read channels. The acquisition of the electrical signals starts when the user presses down the button, acquiring 256 points per second in at most 24 h. It requires about 8 seconds to set the impedance of each skin type. The read-out values are stored in a NOR memory until they are sent to a smartphone via Bluetooth communication. There is an application installed on the smartphone to add the timestamp and the acquired GPS coordinate, and submit the exam to the FTP server. It is possible both to see the recorded exam and monitor cardiac signals in real time. If the file is not successfully sent, an error log is stored, and a new attempt is made after a time x, this is repeated until the exam is submitted to the server. The log is sent to the server along with the next valid exam. The hardware is powered by a rechargeable battery of LI-ION, containing three LEDs and a buzzer to notify the patient of the stage of the examination process and the battery status, a Bluetooth module 4.1 with an antenna, and an USB input for programming and configuring the equipment. The present invention, among all the technological benefits described above, contributes to the immediate diagnosis of symptoms in ACS, AMI, AAF and other cardiac arrhythmias, and allows to dramatically reduce the time between of the first symptoms and the primary angioplasty, definitive treatment of AMI, early initiation of treatment in ACS and AMI and definitive treatment of AAF and other cardiac arrhythmias by proper administration of the drug contained in the airtight compartment (3). The medical and hospital network will have access to the IMS to update data, register new care teams and obtain patient's information that is directed to their service. This allows for less bureaucracy, agility and prevents loss of time in attending to these cardiovascular emergencies, as time is a prime factor in reducing the aggravation of these pathologies, especially when undiagnosed or delayed at the beginning of treatment.

## Claims

1. Monitoring system comprising a wearable system for electrocardiographic monitoring with an airtight container (3) for medicines and integrated medical monitoring system, the wearable system being washable and adapted to recreation, sport and medical use, the wearable system being an ECG vest composed of electrodes (4), electronic circuit and conductive track (2), and the airtight container (3) for specific medicines essential to the treatment of pathologies such as acute coronary syndrome, ACS, acute myocardial infarction, AMI, acute atrial fibrillation, AAF, and other cardiac arrhythmias, coupled to one another, and a mini electrocardiogram, ECG, apparatus containing a Global System Mobile, GSM, modem and Global Positioning System, GPS, or Bluetooth system for connection and information exchange among the devices integrated to a datacenter and a remote monitoring center configured to monitor patients with increased cardiovascular risk, seniors, amateur and professional athletes, and common individuals, being possible to diagnose and treat it as soon as possible, aiming at shortening time of definite treatment of those who present ACS, AMI, AAF, and other cardiac arrhythmias, and the wearable system further comprises a box containing the ECG apparatus and a button, wherein the acquisition of the electrical signals from the ECG is configured to automatically start when the user presses the button down, wherein the airtight container (3) is configured to be controlled by a solenoid and electromechanical system through which a doctor, having an encrypted digital signature, can remotely trigger a door opening of the airtight container containing medicines for proper dispensing after diagnostic confirmation, wherein the GSM modem or Bluetooth system are configured to transfer the data collected by the ECG vest and its location made by GPS online via GSM or by integrating Bluetooth with a smartphone having a specific software or application, app, to a datacenter with an Integrated Monitoring System, IMS, in a secure and reliable manner having 24 × 7 × 365 coverage, and the Integrated Monitoring System is configured to assign the received ECG data to a doctor, who can immediately provide the users with the ECG report in an electronic or remote manner, indicating the first procedures, allowing for an immediate integration to an emergency hospital service and forwarding to users names and addresses of nearby specialized hospitals, wherein remote electrocardiogram monitoring with optional feature of automated interpretation of trace is performed by ECG analysis softwares, supported by a doctor and an expert system, an online software of Integrated Monitoring System, and a software integrated to the smartphone configured to receive electronic signals which enable i) the electronic signals sensed by the electrocardiogram to transmit the trace and to be visible in the smartphone in real time (online), which is enabled by the transmission from the Bluetooth coupled to the electrocardiogram for this smartphone, and ii) the record of this trace over 24 hours.

2. Monitoring system as claimed in claim 1, wherein the wearable system is composed of fabric of polyamide textile yarns or any other fabrics like synthetic and expansible rubbers that may i) offer protection to skin against harmful effects of sun, ii) have antibacterial agents for preventing body odor, iii) be biodegradable, allowing the clothes made therefrom to be decomposed when discarded.

3. Monitoring system as claimed in claim 2, wherein the electrodes (4) are composed of stainless steel yarns horizontally and/or vertically embroidered one another in a crossed or meshed manner, so as to form a rectangle- or square-shaped mesh to widen the surface of contact with the underlying skin, having conductive properties of electronic signals, or are composed of silver- or carbon-based pastes, dyes or conductive stretchable textiles, regardless or singly associated to the mesh of stainless steel yarns or associated to the silver- or carbon-based dye, pastes or conductive stretchable textiles, in addition to serve to print low voltage electronic circuits over elastic films and textile substrates due to contain an encapsulating stretchable component which is used to protect the low voltage circuit printed onto the elastic film and the substrate textile.

4. Monitoring system as claimed in claim 1, wherein the smartphone has an application, app, installed to add time stamp and the coordinate acquired from the GPS, and an exam is passed through the FTP server, and it is possible both to see recorded exams and monitor in real time cardiac signals, and if the file is not successfully sent, it is stored into an error log, and a new attempt will be repeatedly carried out until the exam is passed through the server that is sent to the host together with the next valid exam.

## Patentansprüche

1. Überwachungssystem, umfassend ein tragbares System zur elektrokardiographischen Überwachung mit einem luftdichten Container (3) für Medikamente und integriertem medizinischen Überwachungssystem, wobei das tragbare System gewaschen werden kann und für Freizeit, Sport und medizinische Zwecke eingerichtet ist, wobei das tragbare System eine EKG-Weste ist, die aus Elektroden (4), einer elektronischen Schaltung und einer leitfähigen Bahn (2) besteht, und dem luftdichten Container (3) für spezifische Medikamente, die für die Behandlung von Pathologien wie dem akuten Koronarsyndrom, ACS, dem akuten Herzinfarkt, AMI, dem akuten Vorhofflimmern, AAF, und anderen Rhythmusstörungen unerlässlich sind, miteinander gekoppelt sind, und ein Gerät für ein Minielektrokardiogramm, EKG, das ein Modem des globalen Mobilsystems, GSM, und ein globales Positionierungssystem, GPS, enthält, oder Bluetooth-System für die Verbindung und den Informationsaustausch zwischen den Vorrichtungen, die in ein Datenzentrum und ein entferntes Überwachungszentrum integriert sind, das so konfiguriert ist, dass es Patienten mit erhöhtem kardiovaskulärem Risiko, Ältere, Amateur- und Berufssportler und gewöhnliche Individuen überwachen kann, wobei es möglich ist, diese so schnell wie möglich zu diagnostizieren und zu behandeln, mit dem Ziel, die Zeit der definitiven Behandlung derjenigen zu verkürzen, die ACS, AMI, AAF und andere Herzrhythmusstörungen aufweisen, und das tragbare System ferner einen Kasten umfasst, der das EKG-Gerät und eine Schaltfläche enthält, wobei die Erfassung der elektrischen Signale aus dem EKG so konfiguriert ist, dass sie automatisch beginnt, wenn der Benutzer die Schaltfläche nach unten drückt, wobei der luftdichte Container (3) so konfiguriert ist, dass er durch ein elektromagnetisches und elektromechanisches System gesteuert wird, durch das ein Arzt, der eine verschlüsselte digitale Signatur aufweist, aus der Ferne eine Türöffnung des luftdichten Containers, der Medikamente enthält, zur ordnungsgemäßen Abgabe nach der Diagnosebestätigung auslösen kann, wobei das GSM-Modem oder das Bluetooth-System so konfiguriert sind, dass sie die von der EKG-Weste gesammelten Daten und ihre per GPS ermittelte Stelle online über GSM oder durch die Integration von Bluetooth mit einem Smartphone, das über eine spezifische Software oder Anwendung, App, verfügt, an ein Rechenzentrum mit einem Integrierten Überwachungssystem, IMS, auf sichere und zuverlässige Art und Weise mit 24 × 7 × 365 Abdeckung übertragen, und das Integrierte Überwachungssystem so konfiguriert ist, dass es die zugewiesenen EKG-Daten einem Arzt zuweist, der den Benutzern sofort den EKG-Bericht auf elektronischem Wege oder aus der Ferne bereitstellen kann, wobei die ersten Verfahren angegeben werden, was eine sofortige Einbindung in einen Krankenhausnotdienst und die Weiterleitung von Namen und Adressen von nahegelegenen spezialisierten Krankenhäusern an die Benutzer ermöglicht, wobei die Fernüberwachung des Elektrokardiogramms mit dem optionalen Merkmal der automatischen Interpretation der Kurve durch EKG-Analysesoftware ausgeführt wird, die von einem Arzt und einem Expertensystem, einer Online-Software des integrierten Überwachungssystems und einer in das Smartphone integrierten Software unterstützt wird, die so konfiguriert ist, dass sie elektronische Signale empfängt, die es ermöglichen, dass i) die vom Elektrokardiogramm erfassten elektronischen Signale die Kurve übertragen und im Smartphone in Echtzeit (online) sichtbar sind, was durch die Übertragung von dem mit dem Elektrokardiogramm gekoppelten Bluetooth für dieses Smartphone ermöglicht wird, und ii) die Aufzeichnung dieser Kurve über 24 Stunden.

2. Überwachungssystem nach Anspruch 1, wobei das tragbare System aus einem Gewebe aus Polyamidtextilgarn oder anderen Geweben wie synthetischen und dehnbaren Kautschuken besteht, die i) Schutz für die Haut gegen schädliche Wirkungen der Sonne bieten, ii) antibakterielle Mittel zur Verhinderung von Körpergeruch aufweisen, iii) biologisch abbaubar sind, was die Zersetzung der daraus hergestellten Kleidung ermöglicht, wenn sie weggeworfen wird.

3. Überwachungssystem nach Anspruch 2, wobei die Elektroden (4) aus Edelstahlgarnen bestehen, die horizontal und/oder vertikal kreuz- oder maschenförmig miteinander verwoben sind, um ein rechteckiges oder quadratisches Netz zu bilden, um die Oberfläche des Kontakts mit der darunterliegenden Haut zu verbreitern, das leitende Eigenschaften für elektronische Signale aufweist, oder aus Pasten, Farbstoffen oder leitfähigen dehnbaren Textilien auf Silber- oder Kohlenstoffbasis bestehen, unabhängig oder einzeln dem Netz aus rostfreien Stahlgarnen zugeordnet sind oder den silber- oder kohlenstoffbasierten Farbstoffen, Pasten oder leitfähigen dehnbaren Textilien zugeordnet sind, zusätzlich dazu dienen, elektronische Schaltungen mit niedriger Spannung auf elastische Filme und textile Substrate zu drucken, weil sie eine einkapselnde dehnbare Komponente enthalten, die verwendet wird, um die auf den elastischen Film und das textile Substrat gedruckte elektronische Schaltung mit niedriger Spannung zu schützen.

4. Überwachungssystem nach Anspruch 1, wobei das Smartphone eine Anwendung, App, installiert hat, um den Zeitstempel und die vom GPS erfassten Koordinaten hinzuzufügen, und eine Prüfung durch den FTP-Server geleitet wird, und es möglich ist, sowohl aufgezeichnete Prüfungen zu sehen als auch Herzsignale in Echtzeit zu überwachen, und wenn die Datei nicht erfolgreich gesendet wird, wird sie in einem Fehlerprotokoll gespeichert, und ein neuer Versuch wird wiederholt durchgeführt, bis die Prüfung durch den Server hindurchgeht, der zusammen mit der nächsten gültigen Prüfung an den Host gesendet wird.

## Revendications

1. Système de surveillance comprenant un système portable pour une surveillance électrocardiographique avec un contenant hermétique (3) pour des médicaments et un système intégré de surveillance médicale, le système portable étant lavable et adapté aux loisirs, au sport et à l'utilisation médicale, le système portable étant un gilet ECG composé d'électrodes (4), d'un circuit électronique et d'une piste conductrice (2), et le contenant hermétique (3) pour des médicaments spécifiques essentiels au traitement de pathologies telles que le syndrome coronarien aigu, SCA, l'infarctus aigu du myocarde, IAM, la fibrillation auriculaire aiguë, FAA, et d'autres arythmies cardiaques, couplées l'une à l'autre, et un mini appareil d'électrocardiogramme, ECG, contenant un modem de système mondial pour mobiles, GSM, et un système de positionnement mondial, GPS, ou un système Bluetooth pour la connexion et l'échange d'informations entre les dispositifs intégrés à un centre de données et à un centre de surveillance à distance configuré pour surveiller des patients présentant un risque cardiovasculaire accru, des personnes âgées, des athlètes amateurs et professionnels, et des individus ordinaires, afin de pouvoir diagnostiquer et traiter le plus rapidement possible, dans le but de raccourcir le temps de traitement définitif des personnes présentant un SCA, un IAM, une AAF, et d'autres arythmies cardiaques, et le système portable comprend en outre une boîte contenant l'appareil ECG et un bouton, dans lequel l'acquisition des signaux électriques de l'ECG est configurée pour démarrer automatiquement lorsque l'utilisateur appuie sur le bouton, dans lequel le contenant hermétique (3) est configuré pour être commandé par un solénoïde et un système électromécanique grâce auxquels un médecin, ayant une signature numérique cryptée, peut déclencher à distance l'ouverture d'une porte du contenant hermétique contenant des médicaments pour une distribution appropriée après confirmation de diagnostic, dans lequel le modem GSM ou le système Bluetooth sont configurés pour transférer les données collectées par le gilet ECG et sa localisation par GPS en ligne par le biais du GSM ou en intégrant le Bluetooth à un smartphone doté d'un logiciel ou d'une application, app, spécifique, à un centre de données doté d'un système de surveillance intégré, IMS, d'une manière sécurisée et fiable avec une couverture 24 × 7 × 365, et le système de surveillance intégré est configuré pour attribuer les données ECG reçues à un médecin, qui peut immédiatement fournir aux utilisateurs le rapport ECG par voie électronique ou à distance, en indiquant les premières procédures, permettant une intégration immédiate à un service hospitalier d'urgence et transmettant aux utilisateurs des noms et adresses d'hôpitaux spécialisés proches, dans lequel la surveillance d'électrocardiogramme à distance avec une fonction facultative d'interprétation automatique du tracé est effectuée par des logiciels d'analyse ECG, assistés par un médecin et un système expert, un logiciel en ligne du système de surveillance intégré, et un logiciel intégré au smartphone configuré pour recevoir des signaux électroniques qui permettent i) aux signaux électroniques captés par l'électrocardiogramme de transmettre le tracé et d'être visibles sur le smartphone en temps réel (en ligne), ce qui est rendu possible par la transmission du Bluetooth couplé à l'électrocardiogramme pour ce smartphone, et ii) l'enregistrement de ce tracé sur 24 heures.

2. Système de surveillance selon la revendication 1, dans lequel le système portable est composé d'un tissu de fils textiles en polyamide ou de tout autre tissu comme des caoutchoucs synthétiques et expansibles qui peuvent i) offrir une protection à la peau contre les effets nocifs du soleil, ii) avoir des agents antibactériens permettant de prévenir les odeurs corporelles, iii) être biodégradables, permettant aux vêtements fabriqués à partir de ceux-ci d'être décomposés lorsqu'ils sont mis au rebut.

3. Système de surveillance selon la revendication 2, dans lequel les électrodes (4) sont composées de fils d'acier inoxydable brodés horizontalement et/ou verticalement entre eux de manière croisée ou maillée, de façon à former une maille rectangulaire ou carrée afin d'élargir la surface de contact avec la peau sous-jacente, ayant des propriétés conductrices de signaux électroniques, ou sont composées de pâtes, de colorants ou de textiles extensibles conducteurs, à base d'argent ou de carbone, indépendamment ou individuellement associés à la maille de fils d'acier inoxydable ou associés aux colorants, pâtes ou textiles extensibles conducteurs, à base d'argent ou de carbone, en plus de servir à imprimer des circuits électroniques basse tension sur des films élastiques et des substrats textiles en raison de leur teneur en un composant extensible encapsulant qui est utilisé pour protéger le circuit imprimé basse tension sur le film élastique et sur le substrat textile.

4. Système de surveillance selon la revendication 1, dans lequel le smartphone a une application, app, installée pour ajouter un horodatage et les coordonnées acquises à partir du GPS, et un examen est passé par le serveur FTP, et il est possible à la fois de voir les examens enregistrés et de surveiller en temps réel des signaux cardiaques, et si l'envoi du fichier n'est pas réussi, il est stocké dans un journal d'erreurs, et une nouvelle tentative sera effectuée de manière répétée jusqu'à ce que l'examen soit passé par le serveur qui est envoyé à l'hôte conjointement avec l'examen valide suivant.
